# EUROPEAN PATENT SPECIFICATION

(11) **EP 2 173 397 B1**
(45) Date of publication and mention of the grant of the patent: **18.03.2015**
(21) Application number: 08796680.0
(22) Date of filing: 28.07.2008
(51) Int. Cl.: A61L 29/08, A61L 29/14, A61L 31/10, A61L 31/14

(54) **MEDICAL DEVICE HAVING A LUBRICIOUS COATING WITH A HYDROPHILIC COMPOUND IN AN INTERLOCKING NETWORK**
MEDIZINISCHE VORRICHTUNG MIT SCHMIERBESCHICHTUNG MIT HYDROPHILER VERBINDUNG IN EINEM VERBAND
DISPOSITIF MÉDICAL COMPORTANT UN REVÊTEMENT LUBRIFIÉ AVEC UN COMPOSÉ HYDROPHILE DANS UN RÉSEAU CHEVAUCHANT

(30) Priority: 06.08.2007 US 834164
(43) Date of publication of application: 14.04.2010
(60) Divisional application: 10187031.9
(73) Proprietor: Abbott Cardiovascular Systems Inc., Santa Clara, CA 95054 (US)
(72) Inventor: LIN, Tung-Liang, Temecula California 92592 (US); LEE, Jeong, S., Diamond Bar California 91765 (US); BIAGTAN, Emmanuel, Temecula California 92592 (US); BURKETT, David, Temecula California 92591 (US)
(74) Representative: Boult Wade Tennant
(86) International application number: PCT/US2008/071304
(87) International publication number: WO 2009/045612

(56) References cited:
- US-A- 6 110 483
- US-A1- 2005 055 044
- US-A1- 2005 170 071
- US-A1- 2007 043 160
- DEVINE D M ET AL: "Drug release from a N-vinylpyrrolidinone/acrylic acid lubricious hydrophilic coating" JOURNAL OF MATERIALS SCIENCE, KLUWER ACADEMIC PUBLISHERS, BO, vol. 40, no. 13, 1 July 2005 (2005-07-01), pages 3429-3436, XP019210472 ISSN: 1573-4803

## Description

### BACKGROUND OF THE INVENTION

This invention relates to the field of lubricious hydrophilic coatings for intracorporeal medical devices such as a catheter or guidewire.

The use of a medical devices within a patient may be facilitated by the presence of a lubricious surface on the device. For example, intravascular devices, such as catheters and guidewires, are more easily maneuvered within a patient's vasculature when the friction between the walls of the vessel and the intravascular device is reduced. The friction may be reduced by coating the device with a hydrophilic compound which becomes slippery after adsorbing an appreciable amount of water. Consequently, the hydrophilic coating provides lubricity when the coated device is exposed to aqueous solution, as when the coated device is exposed to water prior to insertion in the patient or to the patient's blood during use. Alternatively, coatings, such as fluoropolymers, and silicone, provide lubricity to the surface of an intracorporeal device without the need for exposure to aqueous solution. However, the degree of lubricity may vary greatly depending on the nature of the lubricious coating. Hydrophilic coatings provide superior lubricity compared to hydrophobic coatings, such as silicone, when tested against a biological tissue countersurface. One lubricious composition suitable for use on a medical device is disclosed in US 2005/0170071. The composition includes at least one alkoxylated acrylate compound having at least two acrylate groups per molecule and at least one second component which provides lubricity.

In addition to lowering the coefficient of friction of the coated device, an effective lubricious coating must strongly adhere to the device surface. The lubricious coating should remain adhered to the device surface during potentially extended periods of storage, as well as in response to abrasive forces encountered during use. Poor adhesive strength is undesirable because the lost coating may be left behind inside the patient during use, with a corresponding decrease in the lubricity of the device. Typically, a trade off exists between a coating's lubricity and the coating's adhesive and cohesive strength, so that attempts to increase the durability of lubricious coatings may inadvertently decrease the lubricity of the coating. Durability is particularly an issue on the surfaces of catheters and guidewires which are subjected to significant rubbing and abrasive forces as the devices are slidably advanced through the patient's tortuous vasculature. Consequently, one difficulty has been providing a highly lubricious coating with long lasting lubricity on a surface of a catheter or guidewire.

It would be a significant advance to provide a highly durable hydrophilic coating on a surface of a medical device to render the device highly lubricious. The present invention satisfies these and other needs.

### SUMMARY OF THE INVENTION

The invention is directed to a medical device having a lubricious coating on at least a section of the medical device, the lubricious coating comprising a network of a hydrophilic compound cross-linked to itself and interlocked with a network of a multifunctional polymerized compound. One aspect of the invention is a method of coating a medical device with the lubricious coating. Additional aspects of the invention are directed to including one or more agents in the coating which provide enhanced adhesion of the coating on the device, or which provide faster hydration of the coating and/or improved lubricity. Additionally, the lubricious coating can be provided with one or more therapeutic or diagnostic agents, and in one embodiment the agent elutes relatively quickly in a concentrated release from the lubricious coating upon hydration of the coating during use of the device.

The lubricious coating comprises the cured reaction product of a solution mixture which is applied onto a surface of the medical device and then cured on the device. The solution mixture is formed by mixing together at least the following components: a multifunctional monomer or polymer network-forming compound, a hydrophilic compound, an adhesion promoter, one or more first cross-linkers for cross-linking the multifunctional monomer or polymer, and one or more second cross-linkers, different than the first cross-linkers, for cross-linking the hydrophilic compound. The first cross-linkers preferentially cross-link the multifunctional monomer or polymer relative to the hydrophilic compound, and the second cross-linkers preferentially cross-link the hydrophilic compound relative to the multifunctional monomer or polymer. In a presently preferred embodiment, the network-forming compound is an oligomer during preparation of the solution mixture. However, it may alternatively be added to the solution mixture as a monomer (prepolymerization) or as a longer chain polymer, such that it may undergo a greater or lesser degree of polymerization on the device depending on whether it is added as a monomer, oligomer, or longer chain polymer. Irrespective of whether or not the network-forming compound is added to the solution mixture in the form of a monomer or a relatively low or high molecular weight polymer, it should be understood that the multifunctional monomer or polymer of the solution mixture is in a polymerized state in the finished coating on the device.

The cross-linkers are preferably photo cross-linkers which initiate the cross-linking reactions in response to irradiation with light (e.g., of the ultraviolet or visible wavelengths). However, thermal initiators, such as peroxides, which respond to increased temperature could be used in an alternative embodiment. Thus, although discussed below primarily in terms of the preferred photo cross-linkers for photo-curing the coating, it should be understood that alternative embodiments may include one or more alternative initiators which react by other mechanisms. The terminology photo cross-linkers should be understood to refer to compounds that work by various mechanisms to cause the network-forming cross-linking, including cross-linking agents that become incorporated into the network, or alternatively, photoinitiators that form radicals that result in the cross-linking reaction.

Applied to the surface of a catheter or guidewire, the lubricious coating maintains its lubricity despite the significant rubbing and abrasive force encountered during use, and in a preferred embodiment prevents or inhibits guidewire hang-up in the catheter lumen caused when agglomerations of blood and contrast increase the frictional resistance between the device surfaces and/or decrease the guidewire clearance. In the absence of the second photo cross-linker, the resulting coating would have a significant amount of the hydrophilic compound noncross-linked and only relatively weakly mechanically contained in the polymer network. Such coatings, which may be referred to as a semi-interpenetrating network (semi-IPN) coating, typically loose significant lubricity relatively quickly compared to the coating of the invention. By including a photo cross-linker specifically for the hydrophilic compound, the resulting coating of the invention preferably provides controlled cross-linking, and facilitates optimizing the curing of the coating to ultimately provide a desired amount of lubricity and durability. For example, the duration of the curing, and the amount of the second photo cross-linker relative to the amount of the hydrophilic compound are selected such that the assembled, sterilized device has a highly lubricious yet durable coating.

While not intending to be bound by theory, it is believed that the coating formulation of the invention allows for the hydrophilic compound to become chemically interlocked by cross-linking to itself (via the second photo cross-linker) to form a true interpenetrating network with the cross-linked polymer, without having the cross-linked polymer chemically (covalently) bond to the hydrophilic compound, for enhanced durability with good lubricity. Thus, it is believed that the hydrophilic compound network and the polymer network, which are chemically formed at the same time in the same mixture, are essentially permanently mechanically interlocked together. The coating is thus unlike a semi-IPN in which a noncross-linked hydrophilic compound is non-permanently mechanically intertwined/contained in a cross-linked polymer, and unlike a coating in which a matrix or underlayer polymer is used to chemically bond to the hydrophilic compound.

In the present invention, the coating includes an adhesion promoter which improves the adhesion of the coating onto a polymeric or metal surface of the medical device. The adhesion promoter provides sufficiently strong adhesion onto the surface of the medical device, to thereby avoid the need for a reactive primer layer underneath the coating on the surface of the medical device.

A method of providing a lubricious coating for a medical device generally comprises preparing a solution mixture of a multifunctional monomer or polymer, a hydrophilic compound, one or more first initiators which preferentially cross-links the monomer or polymer relative to the hydrophilic compound, and one or more second initiators, different than the first initiator, which preferentially cross-links the hydrophilic compound relative to the monomer or polymer, and applying a coating of the solution mixture onto the surface of at least a section of the medical device. The coating of applied solution is then cured, such that the resulting lubricious coating is a network of the hydrophilic compound cross-linked to itself and interlocked with a network of the polymerized multifunctional monomer or polymer.

In a presently preferred embodiment, the hydrophilic compound is a poylvinylpyrrolidone, the second photo cross-linker is a diazido compound, the multifunctional monomer or polymer is an acrylate oligomer, and the adhesion promoter is an acid functionalized acrylate. The resulting coating comprises an acrylate network of the polymerized multifunctional acrylate cross-linked to itself and to the cross-linked acid functionalized acrylate adhesion promoter, and a hydrophilic compound network of the polyvinylpyrrolidone cross-linked to itself by the diazido photo cross-linker, such that the hydrophilic compound network is interlocked with the acrylate network. The coated device can be e-beam or ethylene oxide (EtO) sterilized without significantly decreasing the lubricity or durability of the coating.

The lubricious coating of the invention provides significant and long-lasting lubricity. As a result, when applied to a catheter and/or guidewire, the lubricious coating significantly reduces the frictional forces of the guidewire and the surface of a catheter shaft during advancement or retraction within a patient's body lumen for an extended period of time. These and other advantages of the invention will become more apparent from the following detailed description of the invention.

### DETAILED DESCRIPTION OF THE PREFERRED EMBODIMENTS

In one embodiment of the invention the medical device having a lubricious coating of the invention is a balloon catheter. The balloon catheter generally comprises an elongated catheter shaft having an inflation lumen and a guidewire lumen, and an inflatable balloon on a distal shaft section with an interior in fluid communication with the inflation lumen. An adapter mounted on the proximal end of the catheter shaft provides access to the guidewire lumen and connects to a source of inflation fluid for inflating the balloon. In one embodiment the catheter shaft comprises an outer tubular member having the inflation lumen therein, and an inner tubular member disposed in a lumen of the outer tubular member and having the guidewire lumen therein configured to slidably receive a guidewire. The balloon has a proximal skirt section sealingly secured to the distal end of the outer tubular member, and a distal skirt section sealingly secured to a distal end section of the inner tubular member, and an inflatable section therebetween. The catheter can be advanced within a patient's body lumen, together with the guidewire or slidably advanced over a previously introduced guidewire, to a desired location in the patient's body lumen, and the balloon inflated to perform a medical procedure such as dilatation of a stenosis or expansion of a stent. When used as a stent delivery catheter, a stent is mounted on the balloon for delivery and expansion within the patient's body lumen.

The catheter has at least a section coated with a lubricious coating of the invention, and more specifically has the lubricious coating on at least a section of the shaft.

In one embodiment the lubricious coating is on the outer surface of the outer tubular member (the outer lubricious coating), and on the inner surface of the inner tubular member, and on a distal tip section of the shaft . The outer lubricious coating can be provided on various lengths of the catheter, including on the entire outer length of the catheter from the proximal adapter to the distal-most end of the distal tip section (i.e., along the outer surface of the outer tubular member, the balloon and the distal tip section), or on a shorter length, such that the outer lubricious coating typically extends from the distal-most end of the catheter, proximally for at least about 25 to about 40 cm. For example, in one embodiment, the lubricious coating extends along a 25 to 40 cm portion of the catheter along the outer surface of the distal tip section, the balloon, and only a distal portion of the outer tubular member. If the catheter is used for delivery of a stent, a section of the balloon may be masked during coating, so that the stent can be mounted on a noncoated section of the balloon for good stent retention. The lubricious coating on the inner surface of the inner tubular member may extend along the entire length of the inner tubular member from the proximal to the distal end thereof, or along a shorter length. In embodiments in which the lubricious coating is on the inner surface of the inner tubular member and the coating is photo-cured, the inner tubular member is preferably formed of a polymer transparent to the radiation used to cross-link the coating. In one embodiment the outer surface of the balloon has a coating, typically a lubricious coating, different than the lubricious coating on the shaft, as discussed in more detail below. However, as discussed above, the balloon can additionally or alternatively be coated with the same lubricious coating.

In one embodiment the distal tip section of the shaft, formed by the distal end of the inner tubular member and/or by a soft distal tip member secured to the distal end of the inner tubular member and/or balloon proximal skirt, has the lubricious coating on the outer and the inner surface thereof. However, in alternative embodiments, the lubricious coating is located on just the outer or just the inner surface of the distal tip section. In an alternative embodiment the lubricious coating on the outer surface of the distal tip section is further coated with a second, different lubricious coating, which in one embodiment may be the same lubricious coating that is on the balloon. The lubricious coating is sufficiently durable to remain on the distal tip section during assembly of the catheter, so that in one embodiment, the lubricious coating is provided on the distal tip section of the catheter prior to assembly and processing of the catheter, for example by dip coating or wiping on a distal tip member before it is attached to the inner member and/or balloon. After assembly of the catheter, the second lubricious coating is applied on the balloon and tip. The undercoat of lubricious coating of the invention on the distal tip is provided to minimize variations, and enhance the durability of the lubricity of the distal tip of the fully assembled catheter, which improves the ability of the catheter to cross tight stenoses in the patient's body lumen. In a presently preferred embodiment, the hydrophilic coating applied to the distal tip before it is attached to the catheter is the interlocking network lubricious coating discussed in more detail below, although in alternative embodiments a variety of suitable hydrophilic lubricious coatings including PEO or PVP based coatings can be applied to the distal tip before it is attached to the catheter in accordance with a method of the invention.

Although described as being on the outer tubular member, inner tubular member, and distal tip section of the catheter, it should be understood that the coating can alternatively be applied to fewer areas of the catheter such as just the outer tubular member, or to different areas of the catheter. Thus, the lubricious coating of the invention can be applied to a variety of suitable locations on the catheter. Additionally, the lubricious coating can be applied to a variety of suitable alternative medical devices. For example, the lubricious coating may be applied to a guidewire. The guidewire may comprise a metallic core and coiled wire distal tip, and the coating is preferably along at least a distal section of the guidewire including the floppy distal tip. The guidewire having the lubricious coating of the invention thereon preferably advances and retracts with very low friction force within the guidewire lumen of a catheter.

In one embodiment the guidewire has a polymer layer on an outer surface of the metallic core such that the lubricious coating is on an outer surface of the guidewire polymer layer. In one embodiment, the polymer layer is a polyurethane coating or layer on a stainless steel or NiTi core wire of the guidewire, although the polymer layer can be formed of a variety of polymers including polyolefin, copolyamides, copolyesters or filled polyurethane. Fillers such as tungsten, barium, and bismuth and their compounds in general can be added to enhance radiopacity.

The lubricious coating, on the catheter and/or guidewire, comprises the cured reaction product of a solution mixture comprising a multifunctional monomer or polymer network-forming compound; a hydrophilic compound; an adhesion promoter; one or more first cross-linkers for cross-linking the multifunctional monomer or polymer, which preferentially cross-links the multifunctional monomer or polymer relative to the hydrophilic compound; and one or more second cross-linkers, different than the first cross-linkers, for cross-linking the hydrophilic compound, which preferentially cross-links the hydrophilic compound relative to the multifunctional monomer or polymer. The resulting cured coating on the medical device is a network of the hydrophilic compound cross-linked to itself and interlocked with a network of the cross-linked polymerized multifunctional monomer or polymer.

The multifunctional network-forming compound is preferably a triacrylate oligomer such as a high molecular weight ethoxylated trimethylol propane triacrylate (ETMPTA) (e.g., PHOTOMER® 4158, available from Cognis). The ETMPTA oligomer polymerizes and cross-links during curing to form a network of cross-linked ETMPTA. Alternative cross-linkable polymers (formed from alternative multifunctional monomers or polymers) for forming an interlocking network with the hydrophilic compound include urethane, epoxy, polyester acrylates, and unsaturated polyesters, although a triacrylate, and particularly ETMPTA, is preferred due to its enhanced hydrophilic property, and compatibility with common solvents for good manufacturability. Less preferred is a methacrylate due to the slow reaction and sensitivity to oxygen.

Preferred cross-linkers are photosensitive molecules (photo cross-linkers). Specifically, in the embodiment in which the multifunctional oligomer is a triacrylate, the solution mixture preferably includes mixed first photoinitiators including benozophenone, and a benzil dimethyl ketal such as 2,2-dimethoxy-2-phenyl acetophenone (PHOTOMER® 51, available from Cognis) for photocuring the triacrylate. A variety of mixed first photoinitiators are typically provided, which work by different mechanisms to initiate polymerization and cross-linking of the triacrylate (and acrylates in general) as is generally known. For example, upon irradiation, PHOTOMER®51 undergoes a unimolecular bond cleavage to yield free radicals, while the benezophenone undergoes a bimolecular reaction in the presence of alcohol in which hydrogen abstraction creates hydroxyl (or ketal-type) radicals. However, a variety of suitable first photo cross-linkers can be used which preferentially cross-link the multifunctional polymerized monomer or polymer (e.g., triacrylate oligomer). For example, alternative photoinitiators for cross-linking the triacrylate include 1-hydroxy-cyclohexyl-phenyl-ketone, and 2-hydroxy-2-methyl-1-phenyl-1-propanone, although the preferred photoinitiators provide superior manufacturability due at least in part to good solubility. Ultraviolet, as opposed to visible light, photoinitiation is preferred for faster curing time.

A presently preferred hydrophilic compound is a polyvinylpyrrolidone (PVP, (poly (*N*-vinyl-2-pyrrolidone)), which, when in combination with the second photo cross-linker such as a diazidostilbene (DAS) or derivative thereof, cross-links during curing to form a network of cross-linked PVP. Presently preferred PVPs include PVP K-90 and PVP K-120, available for example from ISP Chemicals, Inc., the K number being significant as it is related to the molecular weight of the PVP. Preferred cross-linkable PVPs have a relatively high molecular weight of greater than about 1,000,000 g/mole for cross-linking to form the desired (lubricious) network. A presently preferred diazidostilbene for preferentially cross-linking the PVP is 4,4'-diazido-2, 2'-stilbene disulfonic acid disodium salt. Other possible diazido based second photo cross-linkers that could be used include diazidostilbene derivatives including those set forth in U.S. Patent No. 5,041,570. Upon irradiation, DAS (a photo cross-linking agent) forms a highly reactive intermediate nitrene group on both ends, and then the nitrene groups on the DAS will react with PVP to form the cross-linked network of PVP. In accordance with the invention, the DAS preferentially cross-links the PVP relative to the multifunctional monomer or polymer network-forming compound (e.g., the triacrylate). That is, the DAS cross-links PVP polymer chains together, substantially without cross-linking the polymer chains of the multifunctional polymerized monomer or polymer. Similarly, the first photo cross-linkers are not expected to cross-link the hydrophilic compound (PVP) of the coating of the invention. Additionally, curing the coating does not cross-link, graft or otherwise chemically bond the hydrophilic compound to the polymerized monomer or polymer, or to the substrate. Thus, although a variety of hydrophilic compounds are well known for use in lubricious coatings for medical devices, in the coating of the invention the hydrophilic compound has a specific initiator which can be added to the solution mixture to preferentially cross-link the hydrophilic compound to itself to a desired degree. Alternative hydrophilic compound-second photo cross-linker combinations that can be used in the coating of the invention include the combination of polyethylene glycol diacrylates (PEGDA) and the photoinitiator 2,2-dimethoxy-2-phenylacetophenone.

The amount of the second cross-linkers provided in the solution mixture relative to the amount of the hydrophilic compound, and the duration of the curing is sufficient to form a three dimensional cross-linked network of the hydrophilic compound, although the hydrophilic compound is cross-linked to a greater or less degree depending on the desired performance characteristics of the lubricious coating. The control provided by the invention over the cross-linking of the hydrophilic compound facilitates creating a desired lubricity and durability which can be tailored for different applications. Thus, PVP that is part of the network in the lubricious coating has a greater or lesser degree of cross-linking. Additionally, some noncross-linked hydrophilic compound (i.e., PVP that is not cross-linked and thus not part of the network) or a noncross-linked secondary hydrophilic compound such as PEO are present in the lubricious coating in some embodiments, for enhanced lubricity at the potential expense of durability. Specifically, network lubricious coatings in which durability and not lubricity was at issue would cross-link the hydrophilic compounds to a greater degree to maximize the durability of the coating at the expense of the lubricity, which may be acceptable in some applications. Additionally, because the cross-linking of the hydrophilic compound is more readily controllable in the lubricious coating of the invention, the amount of cross-linking caused by initially photo-curing the coating on the device can be tailored to compensate for any additional cross-linking that may occur later, as for example when sterilizing the coated device by e-beam or EtO sterilization causes further cross-linking of the coating. In one embodiment, the coated device is e-beam sterilized, and the method of coating the device involves (UV) curing the coating on the device for a relatively short duration which is insufficient to cross-link the compounds to the desired degree (e.g., as determined by performance testing of the coated medical device), and subsequently e-beam sterilizing the coated device such that the compounds further cross-link to the desired degree. Similarly, the amount of photo cross-linkers in the coating can be limited to control the amount of cross-linking caused by the photo-curing.

The solution mixture is formed by combining the multifunctional monomer or polymer, one or more hydrophilic compounds, an adhesion promoter, one or more first cross-linkers, and one or more second cross-linkers together in a single solution (the compounds typically having been first dissolved in a suitable solvent before combining to form the single solution). The solution mixture is then applied to the surface of the catheter shaft and/or guidewire, and it can be applied to the device using a variety of suitable methods including dipping, spraying, wiping the solution on the surface of the catheter or guidewire, or drawing the solution through the guidewire lumen of the catheter. The coating is then typically dried on the device before the curing, and the resulting cured coating has the substantially uniform composition provided by the interlocked networks in a single layer. In one embodiment, an adhesion promoting primer is first coated onto the device and cured, and then the lubricious coating solution mixture is applied onto the cured primer. The cured coating has to be hydrated to render it lubricious for use in a medical procedure. The water induction time, i.e., the time required to hydrate the coating, varies depending on the coating formulation. Thus, the terminology "lubricious coating" as used herein should be understood to refer to the finished coating on the device, either before or after the hydrophilic compound is hydrated to render the coating lubricious for use.

In the present invention, the solution mixture includes an adhesion promoter comprising an acid functionalized acrylate which adheres to a surface of the medical device to improve adhesion of the lubricious coating on the medical device. The preferred adhesion promoter bonds to the surface of the substrate (e.g., the polymer surface of the catheter shaft or the guidewire) and also cross-links to the multifunctional polymerized monomer or polymer. Thus, the first initiators preferably cross-link the adhesion promoter, such that the adhesion promoter is cross-linked to itself and to the cross-linked polymerized multifunctional monomer or polymer in the cured lubricious coating. A presently preferred adhesion promoter is PHOTOMER® 4173, an acid functionalized monoacrylate from Cognis, which bonds to a polymeric (and particularly a polyurethane) substrate layer. Alternative adhesion promoters which could be used include the acid functionalized acrylates PHOTOMER® 4703 and 4846 from Cognis. The adhesion promoter is generally about 0.2% to about 20%, more specifically about 1% to about 2%, by weight of the solution mixture. A reactive primer layer on the device, such as these acid functionalized adhesion promoters (plus a photoinitiator) or other primer compounds such as a urethane acrylate, could additionally or alternatively be used to improve adhesion. With or without the adhesion promoter, the coating of the invention adheres to the surface of the device without requiring that the hydrophilic compound is functionalized or otherwise made to reactively chemically bond to a matrix or substrate polymer.

In one embodiment, the solution mixture includes a secondary hydrophilic compound such as polyethylene oxide (PEO) which is different than the network forming hydrophilic compound (e.g., PVP). The secondary hydrophilic compound is substantially noncross-linked in the lubricious coating. Thus, an initiator which preferentially cross-links the secondary hydrophilic compound is not included in the solution mixture, and curing the coating produces relatively little or no cross-linking of the secondary hydrophilic compound. As a result of being substantially noncross-linked, the secondary hydrophilic compound preferably provides a coating which is, at least initially, more lubricious and/or which has a decreased water induction time (i.e., a quicker response to a hydration procedure). For example, a substantially noncross-linked hydrophilic compound such as polyethylene oxide (PEO) in the coating hydrates relatively quickly. Specifically, combining the first hydrophilic compound such as PVP with the secondary hydrophilic compound such as PEO or polyacrylamide provides a coating that preferably has an improved, fast water induction time after sterilization by e-beam or EtO treatment. Noncross-linked PEO or polyacrylamide preferably compensates for an increase in water induction time of the lubricious coating due to both e-beam and EtO sterilization. A variety of suitable hydrophilic compounds can be used as the secondary hydrophilic compound including PEO, polyacrylamide-co-acrylic acid and polyacrylamide. In one embodiment, a relatively small amount of the secondary hydrophilic compound is present in the coating. For example, in one embodiment, the secondary hydrophilic compound is only about 5% by weight of the amount of PVP in the lubricious coating.

In one embodiment, the solution mixture includes a dissolvable ionic compound (i.e., a salt) such as sodium chloride, and the resulting cured lubricious coating has the salt contained (dissolvably) therein at least prior to the hydration procedure used to hydrate the coating for use. The water induction time is believed to decreased relative to the coating without the salt as a result of the presence of the salt in the cured coating.

In one embodiment, the cured lubricious coating has a therapeutic or diagnostic agent. For example, an agent added to the solution mixture is releasably contained in the cured coating such that as the cured coating swells (hydrates) during use, the agent will elute therefrom. The cured lubricious coating can be provided with a variety of agents. Anti-platelet agents, anti-thrombogenic agents, anti-coagulant agents, anti-inflammatory agents, vasodilator agents, and the like are particularly preferred for adding to the lubricious coating on the balloon, outer member, guidewire, and/or within the guidewire lumen of the catheter shaft. A relatively small molecule agent such as aspirin (acetylsalicyclic acid; acetolsal) is particularly desirable in the lubricious coating because its relatively quick elution time from the lubricious coating provides a concentrated quick dose of the aspirin during the initial introduction and advancement of the catheter and/or guidewire in the patient's body lumen. Although controlled, longer term elution of agents from medical device coatings is a goal of many of prior art coatings, relatively quick, uncontrolled elution of the aspirin from the lubricious coating of the invention is desirable. The concentrated release of the aspirin from the lubricious coating upon hydration of the coating provides an anti-platelet affect during positioning of the catheter in the body lumen, which further reduces guidewire hang-up in the catheter guidewire lumen. Although aspirin has a small molecular weight (e.g., 180 g/mol), alternative agents with larger molecular weights than aspirin can alternatively be used in a coating of the invention, such as Hirudin (about 7,000 g/mol) or Heparin (about 12,000 to about 15,000 g/mol).

The lubricious coating of the invention can be provided with a variety of suitable agents (small or large molecule agents) including anti-restenosis agents, and antiinflammatory, anti-coagulating, or pro-healing drugs. The agent is typically provided by adding it into the solution mixture prior to application onto the device, which is a preferred method due to the good manufacturability, control over the amount and location of the agent on the device, and minimal disruption of the lubricity of the coating. Less preferred methods include swelling the cured coating on the device with a solution of the agent prior to use.

In one embodiment the coating on the balloon is different than the lubricious coating on the shaft. For example, the coating on the balloon may be a lubricious coating which has less lubricity or may contain a different therapeutic agent than the coating on the shaft. In alternative embodiments as discussed above, the same lubricious coating on the shaft is provided on the balloon.

In one embodiment, a lubricious coating on the balloon has a relatively short water induction time (hydrates quickly) and includes an anti-restenosis agent such as everolimus or zotarolimus for treating artery disease and/or preventing restenosis. The agent is well preserved in the agent delivery lubricious coating before balloon inflation, and since the water up-take by the agent delivery lubricious coating occurs quickly, the agent is released immediately as the balloon is inflated, for providing a sufficient dose of the agent at the desired site. Typically, the balloon prior to inflation is folded and thus protects some of the coating within the folds as the catheter is first hydrated and advanced within the blood vessel. In one embodiment, the agent delivery lubricious coating on the balloon is the embodiment of the interlocking network lubricious coating described above having the noncross-linked secondary hydrophilic compound added thereto which provides a quick water induction (e.g., noncross-linked PEO in the interlocking network of cross-linked PVP and cross-linked triacrylate). As discussed above, the agent is preferably added to the solution mixture of the lubricious coating prior to coating of the balloon. The balloon having the agent delivery lubricious coating thereon is then folded or otherwise configured into a low profile configuration for advancement within the patient's body lumen.

In one embodiment, the coating on the balloon is a less lubricious coating than the lubricious coating on the shaft, to prevent or inhibit the inflated balloon from slipping out of the desired treatment location in the patient's body lumen (commonly referred to as "watermelon seeding"). There are a number of alternate approaches to making the coating on the balloon as a less lubricious coating than the lubricious coating on the shaft. For example, a more dilute concentration solution of the same ingredients can be applied on the balloon after the same or more concentrated solution is applied over the shaft and balloon. As another example, a coating comprised of the solution incorporating one hydrophilic polymer (for example PEO) can be applied on the balloon, while a coating comprised of the solution incorporating a different hydrophilic polymer (for example PVP) can be applied on the shaft. As another example, the lubricious coating can comprise the reaction product of a solution mixture of a binding multifunctional oligomer (or monomer or higher molecular weight polymer), a photo cross-linker for cross-linking the binding oligomer, and a hydrophilic compound without a photo cross-linker for preferentially cross-linking the hydrophilic compound of the less lubricious coating. The coating on the balloon can thus be formed of the same component compounds as the coating on the shaft but without the second photo cross-linkers, to result in a less lubricious coating. Although the coating has been described as extending along the entire length of the balloon from the proximal to the distal ends of the balloon, it should be understood that in alternative embodiments, the coating can extend along a shorter length of the balloon or beyond the ends of the balloon.

The following example illustrates a solution mixture for a lubricious coating of the invention. In addition to the specific formulation (with the amount of each component expressed as a weight percent of the solution mixture) used in the following example, the Table also gives example solution weight percent ranges for the components which can be used in making coatings of the invention.

**TABLE**

| Chemical | Specific Weight % (Formulation A) | General Weight % Range Formulations |
|---|---|---|
| Ethanol | 79.63 | about 60 to about 80 |
| Isopropanol (IPA) | 5.53 | about 2 to about 10 |
| Water | 5.53 | about 2 to about 10 |
| PVP K-90 | 6.30 | about 2 to about 10 |
| PEO | 0 | about 0 to about 10 |
| PHOTOMER® 4173 | 1.02 | about 0 to about 5 |
| PHOTOMER® 4158 | 1.89 | about 1 to about 5 |
| PHOTOMER® 51 | 0.019 | about 0.01 to about 0.05 |
| Benzophenone | 0.019 | about 0.01 to about 0.05 |
| 4,4'-diazido-2,2-stilbenedisulfonic acid disodium salt hydrate | 0.063 | about 0.01 to about 0.25 |

A solution mixture of formulation A listed in the Table was applied by dip coating onto a guidewire which had a metallic core wire covered by a polymer layer of a tungsten filled polyurethane polymer. In a testing procedure in which the coated guidewire is repeatedly advanced and retracted within a guidewire lumen of a catheter inner tubular member having an HDPE inner surface (the inner tubular member being filled with sterile water and kept at 37°C with a 31.75mm (1.25") loop), the resulting frictional force caused by the movement of the coated guidewire in the guidewire lumen remained low after multiple cycles, up to 1000 cycles and after twenty four hours. The frictional force after multiple cycles was lower when compared to a guidewire otherwise the same but coated with a lubricious coating of PEO in a cross-linked acrylate (i.e., a solution mixture of isopropanol, water, PEO, trimethylolpropyl triacrylate (TMPTA), hydroxycyclohexyl phenyl ketone and benzophenone, wherein the PEO was a POLYOX WSR N12K and was about 1.6 weight percent of the solution mixture). For example, after thirty cycles, the friction force during pulling or pushing of the guidewire coated with formulation A set forth in the above Table was about 5 grams compared to about 35 to 55 grams for the comparison guidewire.

The dimensions of the catheter are determined largely by the size of the balloon and guidewire to be employed, the catheter type, and the size of the artery or other body lumen through which the catheter must pass or the size of the stent being delivered. Typically, the outer tubular member has an outer diameter of about 0.025 to about 0.04 inch (0.064 to 0.10 cm), usually about 0.037 inch (0.094 cm), and the wall thickness of the outer tubular member can vary from about 0.002 to about 0.008 inch (0.0051 to 0.02 cm), typically about 0.003 to 0.005 inch (0.0076 to 0.013 cm). The inner tubular member typically has an inner diameter of about 0.01 to about 0.018 inch (0.025 to 0.046 cm), usually about 0.016 inch (0.04 cm), and a wall thickness of about 0.004 to about 0.008 inch (0.01 to 0.02 cm). The overall length of the catheter may range from about 100 to about 150 cm, and is typically about 143 cm. Preferably, the balloon has a length about 0.8cm to about 6cm, and an inflated working diameter of about 2 mm to about 10 mm. The guidewire typically has length of about 190 to about 300 cm, and an outer diameter of about 0.010 to about 0.035 inch.

The various catheter components may be joined using conventional bonding methods such as by fusion bonding or use of adhesives. Although the shaft is illustrated as having an inner and outer tubular member, a variety of suitable shaft configurations may be used including a dual lumen extruded shaft having a side-by-side lumens extruded therein. Additionally, although the catheter has been described as an over-the-wire type balloon catheter having a guidewire lumen extending the full length of the catheter, it should be understood that the coating of the invention can be used with a variety of suitable catheters including guiding catheters having a device lumen configured for delivering catheters or other devices, or rapid-exchange type balloon catheters having a guidewire proximal port spaced distally from the proximal end of the catheter shaft.

While the present invention is described herein in terms of certain preferred embodiments, those skilled in the art will recognize that various modifications and improvements may be made to the invention without departing from the scope thereof. For example, although discussed primarily in terms of a coating on a catheter shaft or guidewire, it should be understood that the lubricious coating of the invention can be provided on a variety of medical devices, and is particularly suitable for use on surfaces encountering significant rubbing or abrasive forces during use or assembly and processing. Moreover, although individual features of one embodiment of the invention may be discussed herein in relation to one embodiment and not to other embodiments, it should be apparent that individual features of one embodiment may be combined with one or more features of another embodiment or features from a plurality of embodiments.

## Claims

1. A medical device having at least a section with a lubricious coating that comprises the cured product of a solution mixture applied to the device, the solution mixture comprising:
a) a multifunctional monomer or polymer network-forming compound;
b) a hydrophilic compound;
c) an adhesion promoter;
d) one or more first cross-linkers for cross-linking the multifunctional monomer or polymer, which preferentially cross-links the multifunctional monomer or polymer relative to the hydrophilic compound; and
e) one or more second cross-linkers, different than the first cross-linkers, for cross-linking the hydrophilic compound, which preferentially cross-links the hydrophilic compound relative to the multifunctional monomer or polymer, such that the cured reaction product on the medical device is a network of the hydrophilic compound cross-linked to itself and interlocked with a polymerized network of the monomer or polymer.

2. The device of claim 1 wherein the network-forming compound is a triacrylate.

3. The device of claim 1 wherein the network-forming compound is an ethoxylated trimethylol propane triacrylate oligomer.

4. The device of claim 1 wherein the hydrophilic compound is polyvinylpyrrolidone.

5. The device of claim 1 wherein the hydrophilic compound network is not chemically bonded to the polymerized monomer or polymer network.

6. The device of claim 1 wherein the adhesion promoter is an acid functionalized acrylate.

7. The device of claim 6 wherein the first cross-linkers cross-link the adhesion promoter, such that the adhesion promoter is cross-linked to itself and to the polymerized monomer or polymer in the lubricious coating.

8. The device of claim 1 wherein the first and/or second cross-linkers are photo cross-linkers such that the coating is photo-cured.

9. The device of claim 8 wherein the second photo cross-linker is a diazido compound.

10. The device of claim 9 wherein the diazido compound is a diazidostilbene or a diazidostilbene derivative.

11. The device of claim 8 wherein the first photo cross-linkers are benzophenone and benzil dimethyl ketal.

12. The device of claim 1 wherein the coating includes a secondary hydrophilic compound which is different than the cross-linked hydrophilic compound and which is substantially noncross-linked in the lubricious coating.

13. The device of claim 1 wherein the solution mixture includes a salt, and the salt is dissolvably contained in the cured coating at least prior to hydration of the coating.

14. The device of claim 1 wherein the solution mixture includes a therapeutic agent, such that the networks form in the presence of the therapeutic agent, and the therapeutic agent is releasably contained in the lubricious coating.

15. The device of claim 14 wherein the therapeutic agent is acetylsalicyclic acid.

16. A method of providing a lubricious coating for a medical device, comprising:
a) preparing a solution mixture of a multifunctional monomer or polymer, a hydrophilic compound, an adhesion promoter, one or more first cross-linkers for cross-linking the monomer or polymer, which preferentially cross-links the monomer or polymer relative to the hydrophilic compound, and one or more second cross-linkers, different than the first cross-linkers, for cross-linking the hydrophilic compound, which preferentially cross-links the hydrophilic compound relative to the monomer or polymer; and
b) applying a coating of the solution mixture onto a surface of at least a section of the medical device and curing the coating such that the resulting lubricious coating is a network of the hydrophilic compound cross-linked to itself and interlocked with a polymerized network of the monomer or polymer.

17. The method of claim 16 wherein an amount of the second cross-linkers is limited so that curing the coating cross-links the hydrophilic compound to a lesser degree than desired, and including e-beam or EtO sterilizing the device after b) to further cross-link the hydrophilic compound to a desired degree.

18. The method of claim 16 wherein a duration of the curing is limited so that curing the coating cross-links the hydrophilic compound to a lesser degree than desired, and including e-beam or EtO sterilizing the device after b) to further cross-link the hydrophilic compound to a desired degree.

19. The method of claim 16 wherein curing the coating does not chemically bond the hydrophilic compound to the monomer or polymer.

20. The method of claim 16 wherein the adhesion promoter is an acid functionalized acrylate.

21. The method of claim 20 wherein b) includes cross-linking the adhesion promoter to itself and to the multifunctional polymerized monomer or polymer.

22. The method of claim 16 wherein the solution mixture includes a secondary hydrophilic compound without an initiator which would preferentially cross-link the secondary hydrophilic compound, and wherein curing the coating does not cross-link the secondary hydrophilic compound, such that the secondary hydrophilic compound is noncross-linked in the lubricious coating.

## Patentansprüche

1. Medizintechnische Vorrichtung, die zumindest einen Bereich mit einer gleitfähigen Beschichtung umfasst, die das ausgehärtete Produkt eines auf die Vorrichtung aufgebrachten Lösungsgemisches aufweist, wobei das Lösungsgemisch umfasst:
a) eine multifunktionelle, monomere oder polymere, netzwerkbildende Verbindung,
b) eine hydrophile Verbindung,
c) einen Haftvermittler,
d) ein oder mehrere erste Vernetzungsmittel zum Vernetzen des multifunktionellen Monomers oder Polymers, das bzw. die die multifunktionellen Monomere oder Polymere im Verhältnis zur hydrophilen Verbindung bevorzugt vernetzt bzw. vernetzen, und
e) ein oder mehrere zweite Vernetzungsmittel, die von den ersten Vernetzungsmitteln verschieden sind, zum Vernetzen der hydrophilen Verbindung, das bzw. die die hydrophile Verbindung im Verhältnis zu dem multifunktionellen Monomer oder Polymer bevorzugt vernetzt bzw. vernetzen, sodass das ausgehärtete Reaktionsprodukt auf der medizintechnischen Vorrichtung ein Netzwerk der mit sich selbst vernetzten und mit einem polymerisierten Netzwerk des Monomers oder Polymers verflochtenen hydrophilen Verbindung ist.

2. Vorrichtung nach Anspruch 1, worin es sich bei der netzwerkbildenden Verbindung um ein Triacrylat handelt.

3. Vorrichtung nach Anspruch 1, worin es sich bei der netzwerkbildenden Verbindung um ein ethoxyliertes Trimethylolpropantriacrylat-Oligomer handelt.

4. Vorrichtung nach Anspruch 1, worin es sich bei der hydrophilen Verbindung um Polyvinylpyrrolidon handelt.

5. Vorrichtung nach Anspruch 1, worin das Netzwerk der hydrophilen Verbindung chemisch nicht mit dem polymerisierten Monomer- oder Polymernetzwerk verbunden ist.

6. Vorrichtung nach Anspruch 1, worin der Haftvermittler ein säurefunktionalisiertes Acrylat ist.

7. Vorrichtung nach Anspruch 6, worin die ersten Vernetzungsmittel den Haftvermittler vernetzen, sodass der Haftvermittler mit sich selbst und mit dem polymerisierten Monomer oder Polymer der gleitfähigen Beschichtung vernetzt ist.

8. Vorrichtung nach Anspruch 1, worin es sich bei dem ersten und/oder zweiten Vernetzungsmittel um photosensitive Vernetzungsmittel handelt, sodass die Beschichtung mit Licht ausgehärtet werden kann.

9. Vorrichtung nach Anspruch 8, worin es sich bei dem zweiten Vernetzungsmittel um ein Diazid handelt.

10. Vorrichtung nach Anspruch 9, worin das Diazid ein Diazidostilben oder ein Diazidostilbenderivat ist.

11. Vorrichtung nach Anspruch 8, worin die ersten photosensitiven Vernetzungsmittel Benzophenon und Benzildimethylketal sind.

12. Vorrichtung nach Anspruch 1, worin die Beschichtung eine sekundäre hydrophile Verbindung aufweist, die sich von der vernetzten hydrophilen Verbindung unterscheidet und die in der gleitfähigen Beschichtung im Wesentlich unvernetzt vorliegt.

13. Vorrichtung nach Anspruch 1, worin das Lösungsgemisch ein Salz aufweist und das Salz in der ausgehärteten Beschichtung zumindest vor einer Hydratation der Beschichtung lösbar vorliegt.

14. Vorrichtung nach Anspruch 1, worin das Lösungsgemisch einen therapeutischen Wirkstoff aufweist, sodass sich das Netzwerk in Gegenwart des therapeutischen Wirkstoffs bildet und der therapeutische Wirkstoff in der gleitfähigen Beschichtung freisetzbar enthalten ist.

15. Vorrichtung nach Anspruch 14, worin es sich bei dem therapeutischen Wirkstoff um Actylsalicylsäure handelt.

16. Verfahren zum Herstellen einer gleitfähigen Beschichtung für eine medizintechnische Vorrichtung, wobei das Verfahren aufweist:
a) Herstellen eines Lösungsgemisches aus einem multifunktionellen Monomer oder Polymer, einer hydrophilen Verbindung, einem Haftvermittler, einem oder mehreren ersten Vernetzungsmitteln zum Vernetzen des Monomers oder Polymers, das das bzw. die die multifunktionellen Monomere oder Polymere im Verhältnis zur hydrophilen Verbindung bevorzugt vernetzt bzw. vernetzen, und einem oder mehreren, von den ersten Vernetzungsmitteln verschiedenen zweiten Vernetzungsmitteln zum Vernetzen der hydrophilen Verbindung, das bzw. die die hydrophile Verbindung im Verhältnis zu dem Monomer oder Polymer bevorzugt vernetzt bzw. vernetzen, und
b) Aufbringen des Lösungsgemisches als Beschichtung auf eine Oberfläche der medizintechnischen Vorrichtung in dem zumindest einen Bereich und Aushärten der Beschichtung so, dass die sich ergebende gleitfähige Beschichtung ein Netzwerk der mit sich selbst vernetzten und mit einem polymerisierten Netzwerk des Monomers oder Polymers verflochtenen hydrophilen Verbindung ist.

17. Verfahren nach Anspruch 16, worin der Mengenanteil der zweiten Vernetzungsmittel so limitiert ist, dass ein Aushärten der Beschichtung die hydrophile Verbindung in einem geringeren Grad als beabsichtigt vernetzt, und das ein Sterilisieren der Vorrichtung mit einem Elektronenstrahl oder mit EtO im Anschluss an b) umfasst, um die hydrophile Verbindung bis zum gewünschten Grad weiter zu vernetzen.

18. Verfahren nach Anspruch 16, worin eine Dauer des Aushärtvorgangs so limitiert ist, dass ein Aushärten der Beschichtung die hydrophile Verbindung in einem geringeren Grad als beabsichtigt vernetzt, und das ein Sterilisieren der Vorrichtung mit einem Elektronenstrahl oder mit EtO im Anschluss an b) umfasst um die hydrophile Verbindung bis zum gewünschten Grad weiter zu vernetzen.

19. Verfahren nach Anspruch 16, worin ein Aushärten der Beschichtung die hydrophile Verbindung chemisch nicht an das Monomer oder Polymer bindet.

20. Verfahren nach Anspruch 16, worin der Haftvermittler ein säurefunktionalisiertes Acrylat ist.

21. Verfahren nach Anspruch 20, worin b) ein Vernetzen des Haftvermittlers mit sich selbst und mit dem multifunktionellen polymerisierten Monomer oder Polymer umfasst.

22. Verfahren nach Anspruch 16, worin das Lösungsgemisch eine sekundäre hydrophile Verbindung ohne einen Initiator aufweist, der die sekundäre hydrophile Verbindung bevorzugt vernetzen würde, und worin ein Aushärten der Beschichtung nicht zu einer Vernetzung der sekundären hydrophilen Verbindung führt, sodass die sekundäre hydrophile Verbindung in der gleitfähigen Beschichtung unvernetzt vorliegt.

## Revendications

1. Dispositif médical ayant au moins une section avec un revêtement lubrifiant qui comprend le produit durci d'un mélange en solution appliqué sur le dispositif, le mélange en solution comprenant :
a) un composé formant un réseau de monomère ou polymère multifonctionnel ;
b) un composé hydrophile ;
c) un activateur d'adhérence ;
d) un ou plusieurs premiers agents de réticulation pour la réticulation du monomère ou polymère multifonctionnel, provoquant la réticulation préférentielle du monomère ou polymère multifonctionnel par rapport au composé hydrophile ; et
e) un ou plusieurs seconds agents de réticulation, différents des premiers agents de réticulation, pour la réticulation du composé hydrophile, provoquant la réticulation préférentielle du composé hydrophile par rapport au monomère ou polymère multifonctionnel, de telle sorte que le produit durci de réaction sur le dispositif médical soit un réseau du composé hydrophile réticulé avec lui-même et enchevêtré avec un réseau polymérisé du monomère ou polymère.

2. Dispositif suivant la revendication 1, dans lequel le composé formant un réseau est un triacrylate.

3. Dispositif suivant la revendication 1, dans lequel le composé formant un réseau est un oligomère d'un triacrylate de triméthylolpropane éthoxylé.

4. Dispositif suivant la revendication 1, dans lequel le composé hydrophile est la polyvinylpyrrolidone.

5. Dispositif suivant la revendication 1, dans lequel le réseau de composé hydrophile n'est pas lié chimiquement au réseau de monomère ou polymère polymérisé.

6. Dispositif suivant la revendication 1, dans lequel l'activateur d'adhérence est un acrylate à fonctionnalisation acide.

7. Dispositif suivant la revendication 6, dans lequel les premiers agents de réticulation provoquent la réticulation de l'activateur d'adhérence, de telle sorte que l'activateur d'adhérence soit réticulé avec lui-même et avec le monomère ou polymère polymérisé dans le revêtement lubrifiant.

8. Dispositif suivant la revendication 1, dans lequel les premiers et/ou seconds agents de réticulation sont des agents de photo-réticulation, de telle sorte que le revêtement soit photo-durci.

9. Dispositif suivant la revendication 8, dans lequel le second agent de photo-réticulation est un composé à fonction diazido.

10. Dispositif suivant la revendication 9, dans lequel le composé à fonction diazido est un diazidostilbène ou un dérivé de diazidostilbène.

11. Dispositif suivant la revendication 8, dans lequel les premiers agents de photo-réticulation sont la benzophénone et le benzildiméthylcétal.

12. Dispositif suivant la revendication 1, dans lequel le revêtement comprend un composé hydrophile secondaire qui est différent du composé hydrophile réticulé et qui est substantiellement non réticulé dans le revêtement lubrifiant.

13. Dispositif suivant la revendication 1, dans lequel le mélange en solution comprend un sel, et le sel est présent, d'une manière apte à la dissolution, dans le revêtement durci au moins avant l'hydratation du revêtement.

14. Dispositif suivant la revendication 1, dans lequel le mélange en solution comprend un agent thérapeutique, de telle sorte que les réseaux se forment en présence de l'agent thérapeutique, et l'agent thérapeutique est présent, de manière apte à la libération, dans le revêtement lubrifiant.

15. Dispositif suivant la revendication 14, dans lequel l'agent thérapeutique est l'acide acétylsalicylique.

16. Procédé pour fournir un revêtement lubrifiant pour un dispositif médical, comprenant :
a) la préparation d'un mélange en solution d'un monomère ou polymère multifonctionnel, d'un composé hydrophile, d'un activateur d'adhérence, d'un ou plusieurs premiers agents de réticulation pour la réticulation du monomère ou polymère, provoquant la réticulation préférentielle du monomère ou polymère par rapport au composé hydrophile, et d'un ou plusieurs seconds agents de réticulation, différents des premiers agents de réticulation, pour la réticulation du composé hydrophile, provoquant la réticulation préférentielle du composé hydrophile par rapport au monomère ou polymère ; et
b) l'application d'un revêtement du mélange en solution sur une surface d'au moins une section du dispositif médical et le durcissement du revêtement de telle sorte que le revêtement lubrifiant résultant soit un réseau du composé du hydrophile réticulé avec lui-même et enchevêtré avec un réseau polymérisé du monomère ou polymère.

17. Procédé suivant la revendication 16, dans lequel une quantité des seconds agents de réticulation est limitée de telle sorte que le durcissement du revêtement provoque la réticulation du composé hydrophile à un degré inférieur au degré désiré, et comprenant la stérilisation au moyen d'un faisceau d'électrons ou de EtO du dispositif après b) pour une réticulation supplémentaire du composé hydrophile à un degré désiré.

18. Procédé suivant la revendication 16, dans lequel une durée de durcissement est limitée, de telle sorte que le durcissement du revêtement provoque la réticulation du composé hydrophile à un degré inférieur au degré désiré, et comprenant la stérilisation au moyen d'un faisceau d'électrons ou de EtO du dispositif après b) pour une réticulation supplémentaire du composé hydrophile à un degré désiré.

19. Procédé suivant la revendication 16, dans lequel le durcissement du revêtement ne provoque la liaison chimique du composé hydrophile au monomère ou polymère.

20. Procédé suivant la revendication 16, dans lequel l'activateur d'adhérence est un acrylate à fonctionnalisation acide.

21. Procédé suivant la revendication 20, dans lequel b) comprend la réticulation de l'activateur d'adhérence avec lui-même et avec le monomère ou polymère multifonctionnel polymérisé.

22. Procédé suivant la revendication 16, dans lequel le mélange en solution comprend un composé hydrophile secondaire sans initiateur qui provoquerait la réticulation préférentielle du composé hydrophile secondaire, et dans lequel le durcissement du revêtement ne provoque pas la réticulation du composé hydrophile secondaire, de telle sorte que le composé hydrophile secondaire soit non réticulé dans le revêtement lubrifiant.
